# EUROPEAN PATENT SPECIFICATION

(11) **EP 1 833 441 B1**
(45) Date of publication and mention of the grant of the patent: **04.08.2010**
(21) Application number: 05854678.9
(22) Date of filing: 19.12.2005
(51) Int. Cl.: A61F 9/007

(54) **SURGE DAMPENING IRRIGATION-ASPIRATION TUBING**
STOSSDÄMPFENDER IRRIGATIONS/ASPIRATIONSSCHLAUCH
TUBULURE D'IRRIGATION-ASPIRATION A AMORTISSEMENT DE SAUTES DE PRESSION

(30) Priority: 20.12.2004 US 17581
(43) Date of publication of application: 19.09.2007
(73) Proprietor: BAUSCH & LOMB INCORPORATED, Rochester, New York 14604-2701 (US)
(72) Inventor: PERKINS, James, T., St. Charles, Missouri 63304 (US)
(74) Representative: Bowman, Paul Alan
(86) International application number: PCT/US2005/046009
(87) International publication number: WO 2006/069016

(56) References cited:
- WO-A-99/38549
- US-A- 4 921 477
- US-A- 5 106 367
- US-A- 5 562 612
- US-A1- 2005 080 375

## Description

### Background of the Invention

### 1. Field of the Invention

The present invention is related to irrigation and aspiration tubing for use in ophthalmic surgery and more specifically, is directed to tubing that assists in dampening post occlusion surge.

### 2. Description of Related Art

A common and dangerous occurrence in ophthalmic surgery is post occlusion surge. During ophthalmic surgery, particularly cataract surgery, as the lens is broken-up and emulsified, such as during phacoemulsification, irrigation fluid is constantly infused into the surgical site and the fluid and emulsified tissue are aspirated away from the surgical site through the phaco handpiece. On occasion bits of tissue are larger than the aspiration lumen in the phaco handpiece, which can result in a clogged aspiration line. As long as the aspiration line remains clogged, a negative pressure of vacuum builds-up throughout the aspiration system. Then, after the clog has been removed, the system can experience what is commonly referred to as surge.

Post occlusion surge can cause serious damage to a patient's eye, such as by rupturing a capsular bag and allowing vitreous to leak from the eye's posterior into the eye's anterior chamber or cause irreparable damage to the cornea's endothelial cells.

Many attempts have been made to eliminate or lessen the effects of post occlusion surge and help eliminate or minimize the damage to the eye after a clog has been removed from the aspiration line. During post occlusion surge, the built-up negative pressure can cause all of the fluid and tissue in the eye to be aspirated out in a very quick fashion, causing the eye to collapse and tissue, such as the capsular bag to be torn, which is highly undesirable. In order to prevent this dangerous event, certain schemes such as fluid and air venting of the aspiration line have been implemented. Fluid and air venting cause a reduction in the negative pressure in the aspiration line and therefore, less surge is experienced upon an occlusion break.

Another scheme to reduce post occlusion surge is to provide a separate irrigation source or a secondary irrigation source near the surgical site so that an excess of irrigation fluid is available to be infused into the surgical site upon post occlusion break. Still another attempt to reduce post occlusion surge is to sense the pressure in the aspiration system and stop, slow down, or reverse the aspiration pump upon a certain vacuum level being reached in the aspiration line. By reversing the pump, the aspiration line then, for a short period, acts as an infusion cannula and can actually push the clogging tissue out of and away from the aspiration path.

The document WO 99/38549 discloses a length of tubing according to the preamble of claim 1 and an ophthalmic system comprising such tubing according to the preamble of claim 6.

While all of the prior art schemes and methods for preventing or reducing post occlusion surge have been in varying degrees successful, in which one or more of such schemes are typically employed during cataract surgery, there remains a desire for other post occlusion surge dampening methods. Particularly desirable are inexpensive and easily implemented methods that can reduce the dangerous effects of post occlusion surge.

### Brief Description of Drawings

FIG. 1 is a partial system diagram using irrigation and aspiration tubing in accordance with the present invention;
FIG. 2 is a cross-sectional view of tubing in accordance with the present invention taken along line x-x of FIG. 1;
FIG. 3 is the same cross-sectional view as FIG. 2, wherein the tubing is experiencing an occlusion;
FIG. 4 is a cross-sectional view of the tubing after post occlusion surge; and
FIG. 5 is a partial system diagram using an alternate embodiment of tubing in accordance with the present invention.

### Detailed Description of the Preferred Embodiment

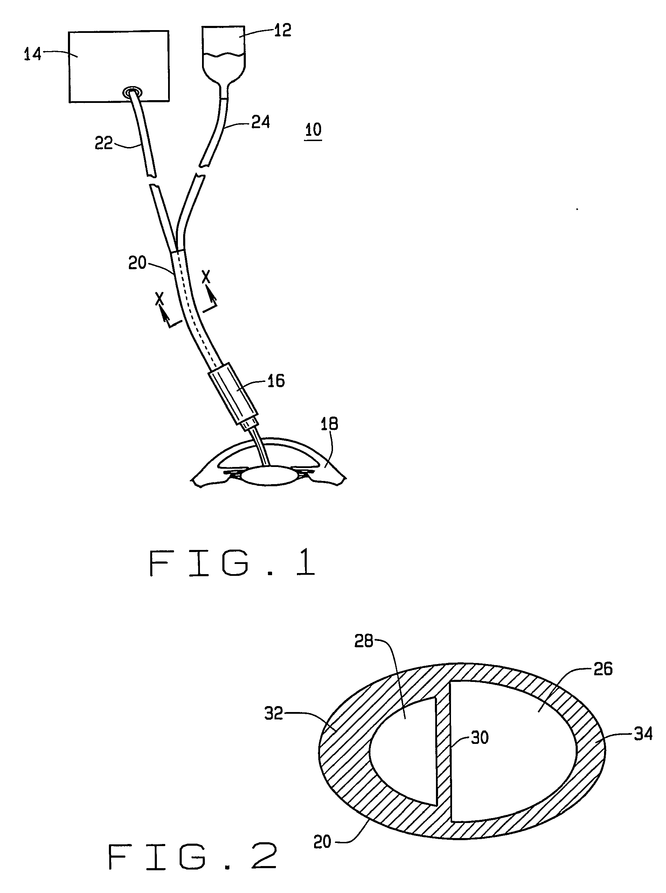

An ophthalmic surgical system 10 includes a source of irrigation fluid 12, such as a balance salt solution (BSS) bottle and an aspiration pumping source 14, which may be a peristaltic or venturi pump or other known pumps for use in ophthalmic surgery. A surgical handpiece 16, such as a phacoemulsification handpiece, infuses irrigation fluid into a patient's eye 18 and aspirates fluids and tissue away from the eye 18. Lengths of tubing 20, 22, and 24 connect the surgical handpiece 16 to each of the irrigation fluid source 12 and the aspiration-pumping source 14. At least a portion of the length of tubing, as designated by the numeral 20, is described in greater detail below, with regard to FIGs. 2 - 4.

Referring to FIG. 2, the length of tubing 20 includes a first irrigation lumen 26 for carrying irrigation fluid from source 12 to the ophthalmic surgical site at eye 18. A second aspiration lumen 28 is also formed within tubing 20 and is shown in FIG. 2 in cross-section. Aspiration lumen 28 carries aspirant from the surgical site at eye 18 to a collection reservoir, not shown.

The irrigation and aspiration lumens 26 and 28 respectively, include a compliant common wall 30, such that any surge occurring after an occlusion break is dampened because of the compliant common wall 30. Wall 30 should be made as thin as possible in order to maintain its at rest shape but yet be compliant enough to deform during occlusion.

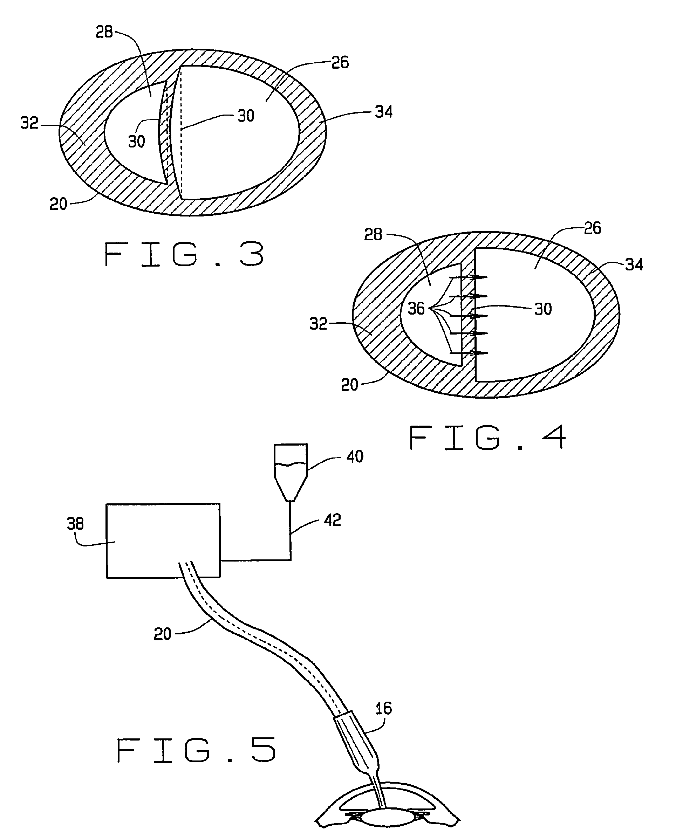

It is also desirable that a portion of the tubing 20 which acts to form the aspiration lumen 28 other than the common wall 30 is thicker than a portion of the tubing forming the irrigation lumen 26, other than the common wall 30, for reducing a compliance of the aspiration lumen 28. As can be seen in FIG. 2, the portion of tubing 20 that forms aspiration lumen 28 is much thicker at 32 than that designated by 34, which surrounds irrigation lumen 26. In this way during the occurrence of an occlusion, wall 30 will experience most of the forces exerted on lumen 28 by the build-up of a vacuum pressure, and therefore, deform as shown in FIG. 3.

FIG. 3 shows a cross-sectional view of tubing 20 which might typically be experienced during occlusion. Wall 30 is shown in its deformed state, wherein lumen 28 essentially becomes smaller and lumen 26 larger. A dashed line showing wall 30 in its resting or non-occluded state is also shown for comparison. As the aspiration lumen 28 builds-up a vacuum, it causes wall 30 to deform and increases the cross-sectional area of irrigation lumen 26 along the length of tubing 20. This increases the volume of liquid available to be introduced into eye 18 upon an occlusion break.

At an occlusion break, wall 30 springs back as indicated by arrows 36 in order to force irrigation liquid very quickly and promptly into the eye 18, thereby reducing any negative effects of post occlusion surge. This is accomplished in a very economical and efficient manner and does not require any additional sensors or monitoring schemes. Rather, by virtue of sharing a common wall 30, the irrigation lumen 26 and the aspiration lumen 28 essentially form a closed loop feedback system, which automatically responds in the correct manner to reduce the undesired effects of post occlusion surge. As can be seen in any FIG. 2 - 4, it is preferred that irrigation lumen 26 be of greater cross-sectional area, and therefore volume than aspiration lumen 28. This disparity in cross-sectional areas helps ensure that enough irrigation fluid is available for delivery to eye 18 upon an occlusion break to sufficiently dampen any post occlusion surge that may occur. It is preferred that tubing 20 be formed of polyvinyl chloride (PVL), though any material suitable for ophthalmic surgical applications is acceptable.

It is also preferred that the tubing 20 be attached directly to surgical handpiece 16 and is of a predetermined length to provide a known amount of dampening. The amount of dampening to be required depends on the surgical settings that the surgeon typically performs cataract surgery at, as well as the type of cataract to be removed, which provides some indication of the likelihood of clogging occurring. Tubing 20 may be of a length less than the total length of tubing running from handpiece 16 to sources 12 and 14, as shown in FIG. 1 or tubing 20 may run the entire length from handpiece 16 to tubing 12 and 14 and only split off as necessary to connect to sources 12 and 14. FIG. 5 shows the tubing 20 running the entire length from handpiece 16 to a surgical console 38. Console 38 is also attached to an irrigation bottle 40 via line 42.

## Claims

1. An irrigation and aspiration tubing length (20,22,24) for use in ophthalmic surgery comprising:
a length of tubing including an irrigation lumen (26) for carrying irrigation fluid from a source (12) to an ophthalmic surgical site (18) and an aspiration lumen (28) for carrying aspirant from the surgical site to a collection reservoir; **characterised in that** the irrigation and aspiration lumens are non-concentric and side-by-side and include a compliant common wall (30), such that any surge occurring after an occlusion break is dampened because of the compliant common wall.

2. The tubing of claim 1, wherein a portion of the tubing forming the aspiration lumen, other than the common wall, is thicker than a portion of the tubing forming the irrigation lumen, other than the common wall, for reducing a compliance of the aspiration lumen.

3. The tubing of claim 2, wherein the irrigation lumen has a larger cross-sectional area compared to an aspiration lumen cross-sectional area.

4. The tubing of claim 1, wherein the tubing is formed of polyvinyl chloride.

5. The tubing of claim 1, wherein the tubing is for attachment directly to a surgical handpiece (16) and is of a predetermined length to provide a known amount of dampening.

6. An ophthalmic surgical system (10) comprising:
a source of irrigation fluid (12);
an aspiration pumping source (14);
a surgical handpiece (16) for infusing irrigation fluid into a patient's eye (18) and for aspirating fluids and tissue away from the eye;
a length of tubing (20,22,24) connecting the surgical handpiece to each of the irrigation fluid source and the aspiration pumping source; and
wherein at least a portion of the length of tubing includes an irritation lumen (26) for carrying irrigation fluid and an aspiration lumen (28) for carrying aspirant fluid and tissue, **characterised in that** the irrigation and aspiration lumens are non-concentric and side-by-side and include a compliant common wall (30), such that any surge occurring after an occlusion break is dampened because of the compliant common wall.

7. The tubing of claim 6, wherein a portion of the tubing forming the aspiration lumen, other than the common wall, is thicker than a portion of the tubing forming the irrigation lumen, other than the common wall, for reducing a compliance of the aspiration lumen,

8. The tubing of claim 7, wherein the irrigation lumen has a larger cross-sectional area compared to an aspiration lumen cross-sectional area.

9. The tubing of claim 6, wherein the tubing is formed of polyvinyl chloride.

10. The tubing of claim 6, wherein the tubing is for attachment directly to a surgical handpiece (16) and is of a predetermined length to provide a known amount of dampening.

## Patentansprüche

1. Irrigations- und Absaugschlauchabschnitt (20, 22, 24) zur Verwendung in der Augenchirurgie, der aufweist:
einen Schlauchabschnitt mit einem Irrigationslumen (26) zum Befördern von Irrigationsfluid von einer Quelle (12) zu einer Augenoperationsstelle (18) und einem Absauglumen (28) zum Befördern von Abgesaugtem von der Operationsstelle zu einem Auffangbehälter; **dadurch gekennzeichnet, daß**
die Irrigations- und Absauglumina nichtkonzentrisch sind und nebeneinander liegen und eine nachgiebige gemeinsame Wand (30) aufweisen, so daß ein etwa nach einem Okklusionsdurchbruch auftretender Druckstoß wegen der nachgiebigen gemeinsamen Wand gedämpft wird.

2. Schlauch nach Anspruch 1, wobei ein nicht zu der gemeinsamen Wand gehörender, das Absauglumen bildender Teil des Schlauchs dicker ist als ein nicht zu der gemeinsamen Wand gehörender, das Irrigationslumen bildender Teil des Schlauchs, um eine Nachgiebigkeit des Absauglumens zu vermindern.

3. Schlauch nach Anspruch 2, wobei das Irrigationslumen eine größere Querschnittsfläche als die Querschnittsfläche des Absauglumens aufweist.

4. Schlauch nach Anspruch 1, wobei der Schlauch aus Polyvinylchlorid geformt ist.

5. Schlauch nach Anspruch 1, wobei der Schlauch zur direkten Befestigung an einem chirurgischen Handstück (16) vorgesehen ist und eine vorgegebene Länge aufweist, um einen bekannten Dämpfungsgrad bereitzustellen.

6. Augenchirurgisches System (10), das aufweist:
eine Irrigationsfluidquelle (12);
eine Absaugpumpquelle (14);
ein chirurgisches Handstück (16) zur Infusion von Irrigationsfluid in das Auge (18) eines Patienten und zum Absaugen von Fluiden und Gewebe aus dem Auge;
einen Schlauchabschnitt (20, 22, 24), der das chirurgische Handstück sowohl mit der Irrigationsfluidquelle als auch mit der Absaugpumpquelle verbindet; und
wobei zumindest ein Teil des Schlauchabschnitts ein Irrigationslumen (26) zum Befördern von Irrigationsfluid und ein Absauglumen (28) zum Befördern von abgesaugtem Fluid und Gewebe aufweist, **dadurch gekennzeichnet, daß** die Irrigations- und Absauglumina nichtkonzentrisch sind und nebeneinander liegen und eine nachgiebige gemeinsame Wand (30) aufweisen, so daß ein etwa nach einem Okklusionsdurchbruch auftretender Druckstoß wegen der nachgiebigen gemeinsamen Wand gedämpft wird.

7. Schlauch nach Anspruch 6, wobei ein nicht zu der gemeinsamen Wand gehörender, das Absauglumen bildender Teil des Schlauchs dicker ist als ein nicht zu der gemeinsamen Wand gehörender, das Irrigationslumen bildender Teil des Schlauchs, um eine Nachgiebigkeit des Absauglumens zu vermindern.

8. Schlauch nach Anspruch 7, wobei das Irrigationslumen eine größere Querschnittsfläche als die Querschnittsfläche des Absauglumens aufweist.

9. Schlauch nach Anspruch 6, wobei der Schlauch aus Polyvinylchlorid geformt ist.

10. Schlauch nach Anspruch 6, wobei der Schlauch zur direkten Befestigung an einem chirurgischen Handstück (16) vorgesehen ist und eine vorgegebene Länge aufweist, um einen bekannten Dämpfungsgrad bereitzustellen.

## Revendications

1. Longueur de tuyau d'irrigation et d'aspiration (20, 22, 24) destinée à être utilisée en chirurgie ophtalmique, comprenant :
une longueur de tuyau comprenant une lumière d'irrigation (26) pour transporter un fluide d'irrigation d'une source (12) jusqu'à un site chirurgical ophtalmique (18) et une lumière d'aspiration (28) pour transporter une substance aspirée du site chirurgical jusqu'à un réservoir de collecte ; **caractérisée en ce que**
les lumières d'irrigation et d'aspiration ne sont pas concentriques et sont côte à côte et comprennent une paroi commune (30) élastique, de sorte que tout afflux survenant après une rupture d'occlusion est amorti du fait de la paroi commune élastique.

2. Tuyau selon la revendication 1, dans lequel une partie du tuyau formant la lumière d'aspiration, autre que la paroi commune, est plus épaisse qu'une partie du tuyau formant la lumière d'irrigation, autre que la paroi commune, pour réduire une élasticité de la lumière d'aspiration.

3. Tuyau selon la revendication 2, dans lequel la lumière d'irrigation a une section transversale plus grande comparée à une section transversale de la lumière d'aspiration.

4. Tuyau selon la revendication 1, dans lequel le tuyau est constitué de polychlorure de vinyle.

5. Tuyau selon la revendication 1, dans lequel le tuyau est destiné à être attaché directement à une pièce à main chirurgicale (16) et a une longueur prédéterminée pour réaliser une quantité connue d'amortissement.

6. Système chirurgical ophtalmique (10) comprenant :
une source de fluide d'irrigation (12) ;
une source de pompage d'aspiration (14) ;
une pièce à main chirurgicale (16) pour infuser un fluide d'irrigation dans l'oeil d'un patient (18) et pour aspirer des fluides et tissus hors de l'oeil ;
une longueur de tuyau (20, 22, 24) reliant la pièce à main chirurgicale à chacune de la source de fluide d'irrigation et de la source de pompage d'aspiration ; et
dans lequel au moins une partie de la longueur de tuyau comprend une lumière d'irrigation (26) pour transporter un fluide d'irrigation et une lumière d'aspiration (28) pour transporter un fluide et un tissu aspirés, **caractérisé en ce que** les lumières d'irrigation et d'aspiration ne sont pas concentriques et sont côte à côte et comprennent une paroi commune (30) élastique, de sorte que tout afflux survenant après une rupture d'occlusion est amorti du fait de la paroi commune élastique.

7. Tuyau selon la revendication 6, dans lequel une partie du tuyau formant la lumière d'aspiration, autre que la paroi commune, est plus épaisse qu'une partie du tuyau formant la lumière d'irrigation, autre que la paroi commune, pour réduire une élasticité de la lumière d'aspiration.

8. Tuyau selon la revendication 7, dans lequel la lumière d'irrigation a une section transversale plus grande comparée à une section transversale de la lumière d'aspiration.

9. Tuyau selon la revendication 6, dans lequel le tuyau est constitué de polychlorure de vinyle.

10. Tuyau selon la revendication 6, dans lequel le tuyau est destiné à être attaché directement à une pièce à main chirurgicale (16) et a une longueur prédéterminée pour assurer une quantité connue d'amortissement.
